# EUROPEAN PATENT APPLICATION

(11) **EP 2 036 587 A1**
(43) Date of publication of application: **18.03.2009**
(21) Application number: 08253054.4
(22) Date of filing: 17.09.2008
(51) Int. Cl.: A61N 1/368

(54) **System for adjusting AV/PV delay**

(30) Priority: 17.09.2007 US 856653
(71) Applicant: PACESETTER, INC., Sylmar, CA 91392-9221 (US)
(72) Inventor: Kristall, Raymond, Winter Park, FL 32789-4466 (US)
(74) Representative: Phillips, Emily Elizabeth

(57) **Abstract**

A system for adjusting AV/PV delay using a pacing device, the system comprising: an implantable pacing device; and a processor configured to: set an initial AV/PV delay for the pacing device; set a threshold for a value related to cardiac function; at least one of monitor a value related to cardiac function and determine a value based on cardiac function; and control the AV/PV delay to lengthen the AV/PV delay when the value related to cardiac function or the value based on cardiac function exceeds the set threshold.

## Description

The present invention relates to medical devices and methods. More specifically, the present invention relates to devices and methods of treating patients with left ventricular dysfunction or congestive heart failure.

Implantable cardiac devices have become increasingly sophisticated and more capable over time. The initial implantable cardiac devices were typically comprised of pacemakers, which provided electrical pacing pulses to the heart at a generally fixed rate. As the technology has developed, more advanced pacing systems have been implanted in patients, which, for example, are capable of providing pacing pulses to the heart only when the pacing system determines that the heart will not provide an intrinsic heart beat. Moreover, such advanced pacemakers are also able to adjust the pacing rate to accommodate different levels of physical activity and corresponding metabolic demand of the patient.

Typically, pacing systems are equipped with sensors, which provide signals that are used by the control unit of the pacing system to determine the pacing rate. Such sensors include activity sensors, such as an accelerometer, metabolic rate sensors, such as a minute ventilation sensor, electrical sensors, such as an impedance sensor, and pressure sensors.

Atrioventricular (AV) delay is the delay between the atrial stimulation pulse and the ventricular stimulation pulse. This delay ensures that the atria are empty before the ventricles contract. It has been documented that patients with left ventricular dysfunction or congestive heart failure (CHF) may require longer AV delays when undergoing exercise. Such longer AV delays may be desirable to allow for adequate filling of the ventricle.

As noted above, AV delay is the delay between the atrial stimulation pulse and the ventricular stimulation pulse. The PV delay is the delay between the intrinsic P-wave (the electrical signal formed through atrial depolarization) and the ventricular stimulation pulse. These may collectively be referred to as AV/PV delay. Embodiments described herein contemplate adjusting AV/PV delay in response to cardiac function.
The invention is set out in the claims.

Embodiments of the present invention contemplate a method for adjusting AV/PV delay in response to cardiac function. In some embodiments, a method of adjusting AV/PV delay using a pacing device may be provided. The method may comprise: setting an initial AV/PV delay for the pacing device; setting a threshold for a value related to cardiac function; determining a value based on cardiac function; and controlling the AV/PV delay to lengthen the AV/PV delay when the value determined based on cardiac function exceeds the set threshold.

The method may also comprise: setting an initial AV/PV delay for the pacing device; setting a threshold for a value related to cardiac function; monitoring a value related to cardiac function; and controlling the AV/PV delay to lengthen the AV/PV delay when the value related to cardiac function exceeds the set threshold.

In some embodiments, controlling of the AV/PV delay may comprise lengthening the AV/PV delay based on at least one of: milliseconds/beats per minute, percentage change/beats per minute, and a hemodynamic and/or physiologic sensor output.

In some embodiments, the controlling of the AV/PV delay may comprise continuing to lengthen the AV/PV delay as the value determined based on cardiac function increases.

Embodiments contemplate using values such as an intrinsic heart rate of a patient, the patient's intrinsic sinus rate, a sensor driven rate, a hemodynamic derived rate, and/or values determined or monitored using a hemodynamic sensor.

In some embodiments, the method may further comprise limiting the lengthening of the AV/PV delay. For example, the lengthening of AV/PV delay may be limited according to a set maximum AV/PV delays. Alternatively or additionally, limiting the lengthening of the AV/PV delay may be based on an output of a hemodynamic and/or physiologic sensor.

Embodiments of the present invention contemplate an implantable medical device or system that is configured to adjust AV/PV delay in response to cardiac function. Such a device or system may be configured to provide pacing to the heart of a patient and thus provide control of AV/PV delay.

In some embodiments, a system for adjusting AV/PV delay using a pacing device may be provided. The system may comprise an implantable pacing device and a processor configured to: set an initial AV/PV delay for the pacing device; set a threshold for a value related to cardiac function; at least one of monitor a value related to cardiac function and determine a value based on cardiac function; and control the AV/PV delay to lengthen the AV/PV delay when the value related to cardiac function or the value based on cardiac function exceeds the set threshold.

In some embodiments, the processor may be configured to lengthen the AV/PV delay based on at least one of: milliseconds/beats per minute, percentage change/beats per minute, and a hemodynamic sensor output.

In some embodiments, the processor may be configured to control the AV/PV delay by continuing to lengthen the AV/PV delay as the value related to cardiac function or the value based on cardiac function increases.

In some embodiments, the system may further comprise a hemodynamic and/or physiologic sensor. In such embodiments, the processor may be configured to monitor the value related to cardiac function or determine the value based on cardiac function using the hemodynamic and/or physiologic sensor.

While multiple embodiments are disclosed, still other embodiments will become apparent to those skilled in the art form the following detailed description, which shows and describes illustrative embodiments. As will be realized, the details provided herein are capable of modifications in various aspects, all without departing form the spirit and scope of the present invention. Accordingly, the drawings and detailed description are to be regarded as illustrative in nature and not restrictive.

FIG. 1 is a simplified, partly cutaway view of a patient's heart and illustrating an implantable stimulation device in electrical communication with at least three leads implanted into the heart for delivering multi-chamber stimulation and shock therapy.

FIG. 2 is a functional block diagram of the multi-chamber implantable stimulation device of FIG. 1, illustrating the basic elements that provide pacing stimulation, cardioversion, and defibrillation in four chambers of the heart.

FIG. 3 is flow chart illustrating a process for adjusting AV/PV delay in response to cardiac function.

The following description is of embodiments presently contemplated for practicing various aspects of the invention. This description is not to be taken in a limiting sense but is made merely for the purpose of describing general principles. The scope of the invention should be ascertained with reference to the issued claims. In the description that follows, like numerals or reference designators will be used to refer to like parts or elements throughout.

Embodiments are described herein in relation to a cardiac stimulation device capable of delivering precisely ordered stimulation pulses to multiple chambers of the heart, referred to herein as multi-chamber stimulation, or to multiple sites within a chamber of the heart, referred to herein as multi-site stimulation. As used herein, the shape of the stimulation pulses is not limited to an exact square or rectangular shape, but may assume any one of a plurality of shapes, which is adequate for the delivery of an energy packet or stimulus.

The stimulation device is intended for use in patients suffering from hemodynamic dysfunction, which may or may not be accompanied by conduction disorders. Precisely controlled stimulation at multiple sites or in multiple chambers is provided to intentionally make use of the pacing function of the heart to improve cardiac hemodynamics by re-coordinating heart chamber contractions and/or preventing arrhythmogenic depolarizations from occurring. Thus, the cardiac stimulation device is capable of delivering at least low-voltage stimulation pulses to multiple stimulation sites for providing pacing therapy, and may include high-voltage stimulation shocks for providing cardioversion therapy and defibrillation therapy.

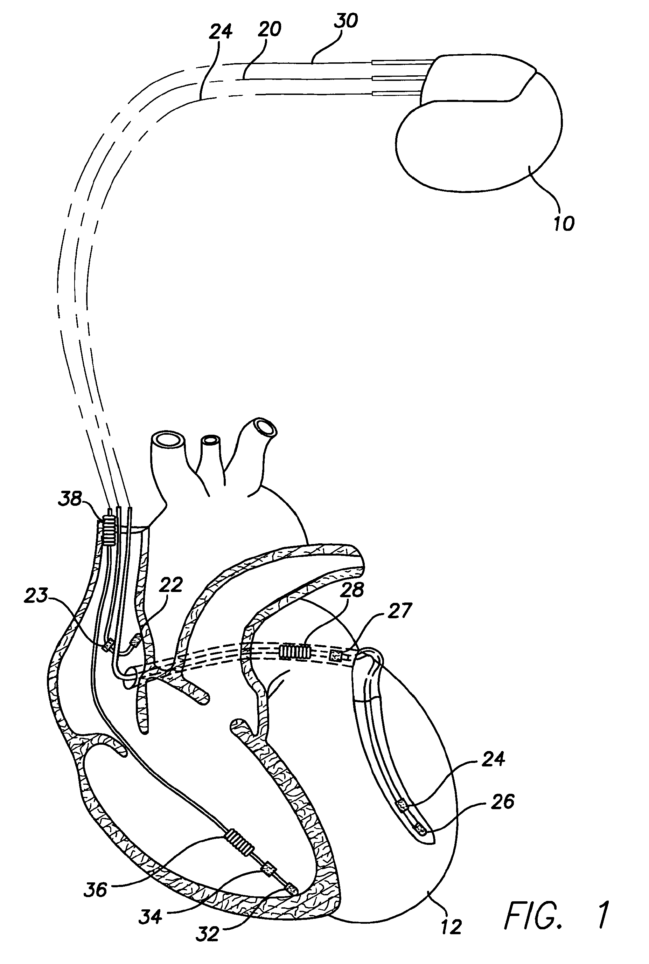

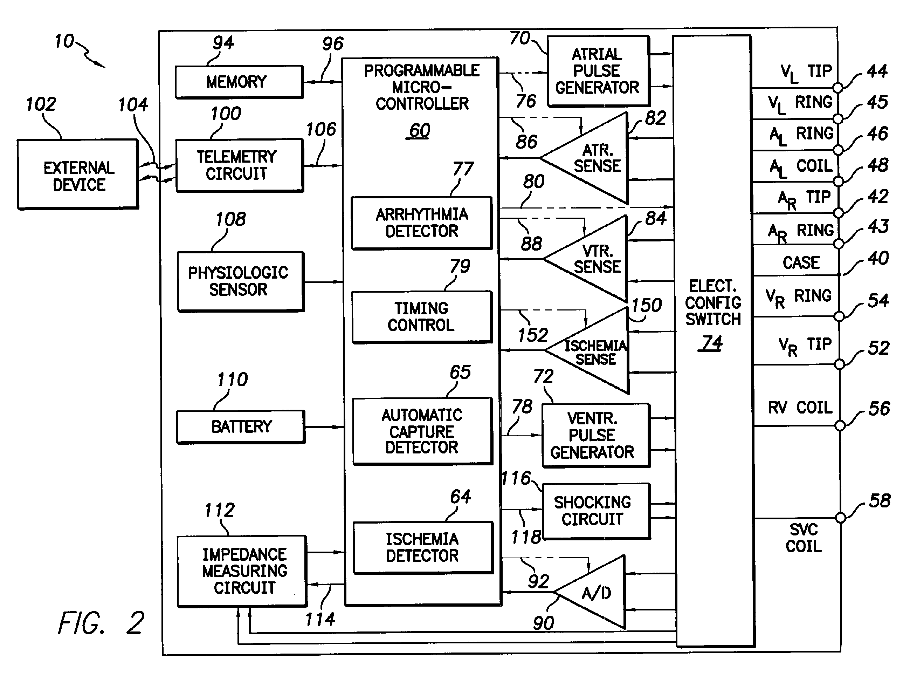

The disclosed device and method are directed at adjusting AV/PV delay. Such adjusting may occur, for example, to optimize or otherwise improve performance of an implantable cardiac device, such as a defibrillator or a pacemaker, providing cardiac stimulation. Thus, the methods described herein may be incorporated in any such cardiac stimulation device. A general cardiac stimulation device will thus be described in conjunction with FIGS. 1 and 2, in which the AV/PV adjustment methods described herein may be implemented. It should be understood, however, that numerous variations exist of such a device in which the methods may be implemented.

FIG. 1 illustrates a stimulation device 10 in electrical communication with a patient's heart 12 via three leads 20, 24 and 30, for example, suitable for delivering multi-chamber stimulation and/or shock therapy. To sense atrial cardiac signals and to provide right atrial chamber stimulation therapy, the stimulation device 10 may be coupled to an implantable right atrial lead 20 including at least an atrial tip electrode 22, which typically is implanted in the patient's right atrial appendage. The right atrial lead 20 may also include an atrial ring electrode 23 to allow bipolar stimulation or sensing in combination with the atrial tip electrode 22.

To sense the left atrial and ventricular cardiac signals and to provide left-chamber stimulation therapy, the stimulation device 10 may be coupled to a "coronary sinus" lead 24 designed to be placed in the "coronary sinus region" via the coronary sinus ostium so as to place at least a distal electrode adjacent to the left ventricle and additional electrode(s) adjacent to the left atrium. As used herein, the phrase "coronary sinus region" refers to the venous vasculature of the left ventricle, including any portion of the coronary sinus, great cardiac vein, left marginal vein, left posterior ventricular vein, middle cardiac vein, and/or small cardiac vein or any other cardiac vein accessible by the coronary sinus.

Accordingly, the coronary sinus lead 24 may be designed to: receive atrial and ventricular cardiac signals; deliver left ventricular pacing therapy using at least a left ventricular tip electrode 26 for unipolar configurations or in combination with left ventricular ring electrode 25 for bipolar configurations; deliver left atrial pacing therapy using at least a left atrial ring electrode 27, and deliver shocking therapy using at least a left atrial coil electrode 28.

The stimulation device 10 is also shown in electrical communication with the patient's heart 12 via an implantable right ventricular lead 30 including, in this embodiment, a right ventricular tip electrode 32, a right ventricular ring electrode 34, a right ventricular (RV) coil electrode 36, and a superior vena cava (SVC) coil electrode 38. Typically, the right ventricular lead 30 is transvenously inserted into the heart 12 so as to place the right ventricular tip electrode 32 in the right ventricular apex so that the RV coil electrode 36 will be positioned in the right ventricle and the SVC coil electrode 38 will be positioned in the right atrium and/or superior vena cava. Accordingly, the right ventricular lead 30 may be capable of receiving cardiac signals and delivering stimulation in the form of pacing and/or shock therapy to the right ventricle.

FIG. 2 illustrates a simplified block diagram of the multi-chamber implantable stimulation device 10, which may be capable of treating both fast and slow arrhythmias with stimulation therapy, including cardioversion, defibrillation, and/or pacing stimulation. The stimulation device 10 may also be capable of automatically adjusting stimulation parameters to provide an optimized cardiac output according to a patient's activity level or metabolic demand and/or for the treatment of congestive heart failure. While a particular multi-chamber device is shown, this is for illustration purposes only, and one of skill in the art could readily duplicate, eliminate or disable the appropriate circuitry in any desired combination to provide a device capable of treating the appropriate chamber(s) with cardioversion, defibrillation and/or pacing stimulation.

The stimulation device 10 may include a housing 40, which is often referred to as a "can", a "case" or a "case electrode", and which may be programmably selected to act as a return electrode for all "unipolar" modes. The housing 40 may further be used as a return electrode alone or in combination with one or more of the coil electrodes 28, 36, or 38, for example, for defibrillation shocking purposes. In accordance with one embodiment, the housing 40 may be used as the return electrode during sensing of intracardiac electrogram (EGM) signals, which may be used for the adjusting methods as described herein.

The housing 40 may further include a connector with a plurality of terminals 42, 43, 44, 45, 46, 48, 52, 54, 56, and 58 (shown schematically and, for convenience, the names of the electrodes to which they are connected are shown next to the corresponding terminals). As such, to achieve right atrial sensing and/or stimulation, the connector may include at least a right atrial tip terminal (A_{R} TIP) 42 adapted for connection to the atrial tip electrode 22. The connector may also include a right atrial ring terminal (A_{R} RING) 43 for connection to the right atrial ring electrode 23.

To achieve left chamber sensing, pacing, and/or shocking, the connector may include at least a left ventricular tip terminal (V_{L} TIP) 44, a left ventricular ring terminal (V_{L} RING) 45, a left atrial ring terminal (A_{L} RING) 46, and a left atrial shocking coil terminal (A_{L} COIL) 48, which are adapted for connection to the left ventricular tip electrode 26, the left ventricular ring electrode 25, the left atrial ring electrode 27, and the left atrial coil electrode 28, respectively.

To support right ventricular sensing, pacing and/or shocking, the connector further may include a right ventricular tip terminal (V_{R} TIP) 52, a right ventricular ring terminal (V_{R} RING) 54, a right ventricular shocking coil terminal (RV COIL) 56, and an SVC shocking coil terminal (SVC COIL) 58, which are adapted for connection to the right ventricular tip electrode 32, right ventricular ring electrode 34, the RV coil electrode 36, and the SVC coil electrode 38, respectively.

At the core of the stimulation device 10 may be a programmable microcontroller 60 that is configured to control the various modes of sensing and/or stimulation therapy. The microcontroller 60 may include a microprocessor, or equivalent control circuitry, designed specifically for controlling the sensing or delivery of stimulation therapy, and may further include RAM or ROM memory, logic and timing circuitry, state machine circuitry, and I/O circuitry. Typically, the microcontroller 60 includes the ability to process or monitor input signals (data) as controlled by a program code stored in a designated block of memory. Any suitable microcontroller 60 may be used that carries out the functions described herein.

FIG. 2 illustrates an atrial pulse generator 70 and a ventricular pulse generator 72 that are configured to respectively generate stimulation pulses for delivery by the right atrial lead 20, the right ventricular lead 30, and/or the coronary sinus lead 24 via a switch 74. It should be understood that to provide stimulation therapy in each of the four chambers of the heart, the atrial pulse generator 70 and the ventricular pulse generator 72 may include dedicated, independent pulse generators, multiplexed pulse generators, or shared pulse generators. The atrial pulse generator 70 and the ventricular pulse generator 72 may be controlled by the microcontroller 60 via appropriate control signals 76 and 78, respectively, to trigger or inhibit the stimulation pulses.

The microcontroller 60 may further include timing control circuitry 79, which may be configured to control the timing of such stimulation pulses (e.g., pacing rate, atrio-ventricular (AV) delay, atrial interchamber (A--A) delay, or ventricular interchamber (V--V) delay, etc.), as well as to keep track of the timing of refractory periods, noise detection windows, evoked response windows, alert intervals, marker channel timing, etc. Thus, the microcontroller 60 may be configured to control AV/PV delay.

The switch 74 may include a plurality of switches for connecting the desired electrodes to the appropriate I/O circuits, thereby providing complete electrode programmability. Accordingly, the switch 74, in response to a control signal 80 from the microcontroller 60, may determine the polarity of the stimulation pulses (e.g., unipolar, bipolar, cross-chamber, etc.) by selectively closing the appropriate combination of switches.

Atrial sensing circuits 82 and ventricular sensing circuits 84 may also be selectively coupled to the right atrial lead 20, coronary sinus lead 24, and the right ventricular lead 30, through the switch 74, for detecting the presence of cardiac activity in each of the four chambers of the heart. Accordingly, the atrial and ventricular sensing circuits 82 and 84 may include dedicated sense amplifiers, multiplexed amplifiers, or shared amplifiers. The switch 74 may determine the "sensing polarity" of the cardiac signal by selectively closing the appropriate switches. In this way, the clinician may program the sensing polarity independent of the stimulation polarity.

Each of the atrial sensing circuit 82 or the ventricular sensing circuit 84 may employ one or more low power, precision amplifiers with programmable gain and automatic gain or sensitivity control, bandpass filtering, and a threshold detection circuit, to selectively sense a cardiac signal of interest. The automatic sensitivity control may enable the stimulation device 10 to deal effectively with the difficult problem of sensing the low amplitude signal characteristics of atrial or ventricular fibrillation.

The outputs of the atrial and ventricular sensing circuits 82 and 84 may be connected to the microcontroller 60 for triggering or inhibiting the atrial and ventricular pulse generators 70 and 72, respectively, in a demand fashion, in response to the absence or presence of cardiac activity, respectively, in the appropriate chambers of the heart (i.e., pacing and/or defibrillation). The atrial and ventricular sensing circuits 82 and 84, in turn, may receive control signals over signal lines 86 and 88 from the microcontroller 60, for controlling the gain, threshold, polarization charge removal circuitry, and the timing of any blocking circuitry coupled to the inputs of the atrial and ventricular sensing circuits 82 and 84.

The stimulation device 10 may further include an ischemia detector 64 dedicated to sensing EGM signals that are evaluated for changes indicative of myocardial ischemia, which may be included in the microcontroller 60. Although not described in detail below, and not shown in FIGS. 1 and 2, it should be understood that electrocardiogram (ECG) signals may also be sensed using surface electrodes (e.g., epicardial electrodes) connected to the heart 12. As such, such surface electrodes may be included in the possible combinations. For the sake of clarity and simplicity, however, only the intracardial electrodes are illustrated in FIGS. 1 and 2.

For arrhythmia detection, the stimulation device 10 may include an arrhythmia detector 77 that utilizes the atrial and ventricular sensing circuits 82 and 84 to sense cardiac signals, for determining whether a rhythm is physiologic or pathologic. Arrhythmia detection may be performed as is known in the art.

Cardiac signals may be applied to the inputs of a data acquisition system 90, which is depicted as an analog-to-digital (A/D) converter for simplicity of illustration. The data acquisition system 90 may be configured to acquire EGM signals (and/or ECG signals as noted above), convert the raw analog data into digital signals, and store the digital signals for later processing and/or telemetric transmission to an external device 102. The data acquisition system 90 may be coupled to the right atrial lead 20, the coronary sinus lead 24, and the right ventricular lead 30 through the switch 74 to sample cardiac signals across any pair of desired electrodes.

Advantageously, the data acquisition system 90 may be coupled to the microcontroller 60 or another detection circuitry, for detecting an evoked response from the heart 12 in response to an applied stimulus, thereby aiding in the detection of "capture". In the embodiment shown in FIG. 2, the microcontroller 60 may include an automatic capture detector 65 that searches for an evoked response signal following a stimulation pulse during a "detection window" set by timing control circuitry 79.

The microcontroller 60 may enable the data acquisition system 90 via control signal 92 to sample the cardiac signal that falls in the capture detection window. The sampled signal may be evaluated by an automatic capture detector 65 to determine if it is an evoked response signal based on its amplitude, peak slope, morphology or another signal feature or combination of features. The detection of an evoked response during the detection window indicates that capture has occurred.

The microcontroller 60 may further be coupled to a memory 94 by a suitable data/address bus 96, wherein the programmable operating parameters used by the microcontroller 60 are stored and modified, as required, to customize the operation of the stimulation device 10 to suit the needs of a particular patient. Such operating parameters may define, for example, stimulation pulse amplitude, pulse duration, electrode polarity, rate, sensitivity, automatic features, arrhythmia detection criteria, and the amplitude, waveshape and vector of each stimulation pulse to be delivered to the patient's heart 12 within each respective tier of therapy. Further, such operating parameters may define AV/PV delay adjustment criteria and/or configurations for adjusting AV/PV delay.

Advantageously, the operating parameters of the stimulation device 10 may be non-invasively programmed into the memory 94 through a telemetry circuit 100 in telemetric communication with the external device 102, such as a programmer, transtelephonic transceiver, or a diagnostic system analyzer. The telemetry circuit 100 may be activated by the microcontroller 60 by a control signal 106. The telemetry circuit 100 advantageously may allow intracardiac electrograms and status information relating to the operation of the stimulation device 10 (as contained in the microcontroller 60 or memory 94) to be sent to the external device 102 through an established communication link 104.

The stimulation device 10 may further include a physiologic sensor 108, commonly referred to as a "rate-responsive" sensor because it is typically used to adjust stimulation rate according to the exercise state of the patient. However, the physiological sensor 108 may further be used to detect changes in cardiac output, changes in the physiological condition of the heart, or diurnal changes in activity (e.g. detecting sleep and wake states), which may be used to adjust the various stimulation parameters, for example. In embodiments, the physiological sensor may be a hemodynamic sensor.

The stimulation device 10 may additionally include a power source such as a battery 110 that provides operating power to all the circuits shown in FIG. 2. As further illustrated in FIG. 2, the stimulation device 10 may include an impedance measuring circuit 112, which may be enabled by the microcontroller 60 by control signal 114. Known uses for an impedance measuring circuit 112 include, but are not limited to, lead impedance surveillance during the acute and chronic phases for proper lead positioning or dislodgment; detecting operable electrodes and automatically switching to an operable pair if dislodgment occurs; measuring respiration or minute ventilation; measuring thoracic impedance for determining shock thresholds; detecting when the device has been implanted; measuring stroke volume; and detecting the opening of heart valves, etc. The impedance measuring circuit 112 advantageously may be coupled to the switch 74 so that any desired electrode may be used.

If it is a function of the stimulation device 10 to operate as an implantable cardioverter/defibrillator (ICD) device, it must detect the occurrence of an arrhythmia, and automatically apply an appropriate electrical stimulation or shock therapy to the heart aimed at terminating the detected arrhythmia. To this end, the microcontroller 60 may further control a shocking circuit 116 via a control signal 118. The shock therapy may be applied to the patient's heart through at least two shocking electrodes, and as shown in this embodiment, selected from the left atrial coil electrode 28, the RV coil electrode 36, and/or the SVC coil electrode 38 (FIG. 1). As noted above, the housing 40 may act as an active electrode in combination with the RV coil electrode 36, or as part of a split electrical vector using the SVC coil electrode 38 or the left atrial coil electrode 28.

The electrode configuration switch 74 illustrated in FIG. 2 may allow various electrode combinations to be used for stimulation and/or sensing. Thus, various configurations may be defined to control adjustment of AV/PV delay, for example, based on values sensed using one or more of the electrodes.

In embodiments, the goal may be a rate-responsive AV/PV delay. In such embodiments, AV/PV delay may be controlled by lengthening the AV/PV delay once a parameter or value, such as a rate, increases above or exceeds a threshold, such as a rate responsive trigger.

Such an approach may monitor or determine cardiac function, for example, using the patient's intrinsic rate, a sensor driven rate, a hemodynamic derived rate or output of a hemodynamic and/or physiologic sensor, and then adjust the AV/PV delay by lengthening the AV/PV delay. The lengthening may be implemented as milliseconds/beats per minute, percentage change/beats per minute, a function of hemodynamic sensor output, or any other suitable approach designed to ensure that sufficient time (delay) is provided for filling of the ventricle.

The lengthening of the AV/PV delay may continue, for example, as the parameter or value continues to increase. Further, the lengthening of the AV/PV delay may be limited by a parameter, such as a maximum AV/PV delay, or based on hemodynamic sensor output.

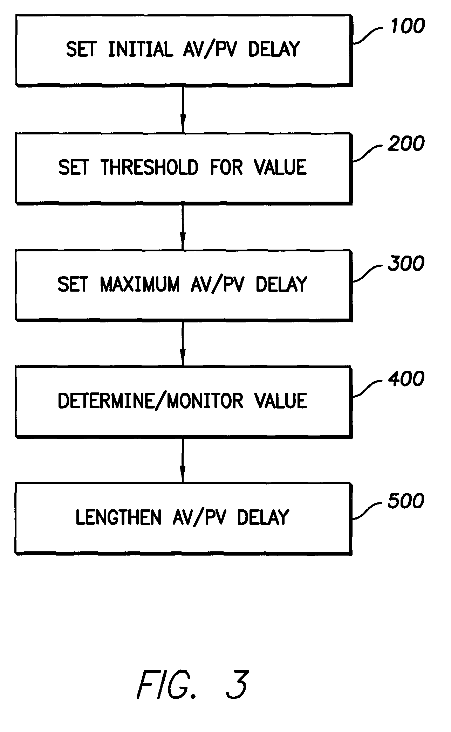

For example, as outlined in FIG. 3, an initial AV/PV delay for a pacing device may be set [BLOCK 100]. The initial AV/PV delay may be set based on various parameters of the patient including, for example, the patient's age, sex, physical condition, health history, as well as based on any cardiac data collected by the pacing device, such as hemodynamic data. In embodiments, the initial AV/PV delay setting may be arbitrarily selected, for example, based on the patient's sex, age, physical condition and/or health history. Alternatively or additionally, the initial AV/PV delay may be set based on electrical optimization (e.g., QuickOpt™), hemodynamic sensor measurements and/or physiologic sensor measurements. Further, the initial AV/PV delay may be a single value or may be a plurality of values set for corresponding cardiac parameters, such as heart rates. For example, the initial AV/PV delay may comprise a table of preset values that are implemented as the corresponding cardiac parameters are sensed.

A threshold for a value related to cardiac function may also be set [BLOCK 200]. The value may be, for example, a rate, such as the patient's intrinsic heart rate, the patient's intrinsic sinus rate, a sensor driven rate (e.g., output of an activity sensor that detects patient activity and allows augmentation of the pacing rate based thereon), a hemodynamic derived rate (e.g., a rate computed from hemodynamic data collected by the pacing device), or may be a value determined using a hemodynamic or physiologic sensor, which may determine other parameters, such as pressure, flow/stroke volume, oxygen saturation, etc., that may indicate any effects on cardiac performance and indicate that an adjustment to the AV/PV delay may be appropriate.

Optionally, the method may involve limiting the adjustment (lengthening) of the AV/PV delay that is possible. For example, a maximum AV/PV delay may be set [BLOCK 300]. Alternatively or additionally to setting a maximum AV/PV delay, the AV/PV delay may be limited based on output of a hemodynamic sensor. Such limitation(s) on the AV/PV delay that may be implemented may help to prevent undesirable risks to the patient and/or the patient's heart. Too long of an AV/PV delay may, for example, contribute to inadequate fill or valvular insufficiency.

Once the threshold is set, a value based on cardiac function may be determined or a value related to cardiac function may be monitored [BLOCK 400] to determine whether the threshold is exceeded. The determination or monitoring may be implemented by a microcontroller, as discussed above, or by any suitable processor configured to provide such function.

The determination or monitoring may be implemented by one or more sensors capable of sensing the desired value. For example, if the patient's intrinsic heart rate is to be monitored, a rate sensor may be included in the pacing device. If a hemodynamic value is to be employed, a suitable hemodynamic sensor may be included in the pacing device. Similarly, if a hemodynamic derived rate is to be employed, one or more hemodynamic sensors may provide data to the processor (microcontroller) to compute the rate.

If/when the threshold is exceeded, the AV/PV delay may be lengthened by the processor (microcontroller) [BLOCK 500]. Such lengthening may be configured to provide sufficient time for filling of the ventricle. In particular, the lengthening may be based on output from one or more hemodynamic sensors. Further, the lengthening may be implemented based on the determined/monitored rate, for example, as milliseconds/beats per minute or percentage change/beats per minute.

For example, if the initial AV/PV delay is a single value, the initial AV/PV delay may be increased (lengthened) once the threshold is exceeded. The AV/PV delay may be adjusted by a single increase, which may be preset, for example, based on the patient's medical history, or may be determined based on current cardiac function, such as based on a rate or hemodynamic parameter(s). Alternatively, once the threshold is exceeded, adjustment of the AV/PV delay may be continuous or periodic based on current cardiac function. For example, the AV/PV delay may continue to be lengthened as the determined/monitored value continues to increase.

If the initial AV/PV delay is a plurality of preset values, then the preset values may be lengthened once the threshold has been exceeded. This may be implemented, for example, where the plurality of preset values is based on one monitored/determined value and the threshold and the lengthening are based on a different monitored/different value. In particular, where the plurality of preset values is based on the patient's heart rate, the threshold and the lengthening may be based on output from one or more hemodynamic sensors. In such case, the AV/PV delay may be dynamically based on both the patient's heart rate and hemodynamic parameter(s). This may help to avoid making changes to the AV/PV delay based on a false reading or potentially a tachycardia.

The AV/PV delay may thus be controlled to provide optimal or otherwise improved cardiac performance via the pacing device. Such control may allow the pacing device to adapt to changing cardiac demands of the patient, for example, as the patient goes from rest to activity or otherwise increases physical effort. In particular, this may improve a cardiac patient's quality of life by improving pacing device performance and may also improve the patient's exercise capacity.

Further, some embodiments contemplate a dynamic adjustment of the AV/PV delay, i.e., both increasing and decreasing the AV/PV delay. For example, an increase in the AV/PV delay may occur in response to activity or exercise by the patient. Once the patient returns to rest and/or a hemodynamic sensor and/or a physiologic sensor measure a parameter value that indicates that the AV/PV delay extension is no longer needed or appropriate, the AV/PV may be decreased toward the initial AV/PV delay setting.

The AV/PV delay may be decreased to the initial AV/PV setting, for example. It should be understood, however, that the decrease in the AV/PV delay may be incremental and/or may be at a predetermine rate. The increments and or the particular rate of decrease may be predetermined, for example, based on the patients characteristics (as discussed above), or may be determined based on one or more measured hemodynamic and/or physiologic parameter(s). Also, it should be understood that the decrease in the AV/PV delay may be to an AV/PV delay other than the initial value, for example, as determined based on one or more measured hemodynamic and/or physiologic parameter(s).

Although the present invention has been described with reference to preferred embodiments, persons skilled in the art will recognize that changes may be made in form and detail without departing from the scope of the invention.

## Claims

1. A system for adjusting AV/PV delay using a pacing device, the system comprising:
an implantable pacing device; and
a processor configured to:
set an initial AV/PV delay for the pacing device;
set a threshold for a value related to cardiac function;
at least one of monitor a value related to cardiac function and determine a value based on cardiac function; and
control the AV/PV delay to lengthen the AV/PV delay when the value related to cardiac function or the value based on cardiac function exceeds the set threshold.

2. The system of claim 1, wherein the processor is configured to lengthen the AV/PV delay based on at least one of: milliseconds/beats per minute, percentage change/beats per minute, and a hemodynamic sensor output.

3. The system of claim 1, wherein the processor is configured to control the AV/PV delay by continuing to lengthen the AV/PV delay as the value related to cardiac function or the value based on cardiac function increases.

4. The system of claim 1, wherein the value based on/related to cardiac function comprises an intrinsic heart rate of a patient.

5. The system of claim 4, wherein the intrinsic heart rate of the patient comprises the patient's intrinsic sinus rate.

6. The system of claim 1, wherein the value based on/related to cardiac function comprises a sensor driven rate.

7. The system of claim 1, wherein the value based on/related to cardiac function comprises a hemodynamic derived rate.

8. The system of claim 1, further comprising a hemodynamic sensor, the processor being configured to monitor the value related to cardiac function or determine the value based on cardiac function using the hemodynamic sensor.

9. The system of claim 1, wherein the processor is configured to limit the lengthening of the AV/PV delay.

10. The system of claim 9, wherein the processor is configured to limit the lengthening of the AV/PV delay to a maximum AV/PV delay.

11. The system of claim 9, wherein the processor is configured to limit the lengthening of the AV/PV delay based on an output of a hemodynamic sensor.

12. The system according to claim 11, wherein the system is arranged such that the hemodynamic sensor is used to determine the value based on/related to cardiac function.

13. The system of claim 1, wherein the processor is configured to control the AV/PV delay to shorten the lengthened AV/PV delay based on a measured parameter related to cardiac function.
